(19) **Europäisches Patentamt / European Patent Office / Office européen des brevets**

(11) **EP 4 624 567 A1**

(12) # EUROPEAN PATENT APPLICATION
published in accordance with Art. 153(4) EPC

(43) Date of publication:
**01.10.2025 Bulletin 2025/40**

(21) Application number: **23894794.9**

(22) Date of filing: **05.10.2023**

(51) International Patent Classification (IPC):
*C12N 1/20* (2006.01)    *C08J 11/10* (2006.01)
*B09B 3/60* (2022.01)    *C12R 1/01* (2006.01)

(52) Cooperative Patent Classification (CPC):
**B09B 3/60; C08J 11/10; C12N 1/20;** C12R 2001/01;
Y02W 30/62

(86) International application number:
**PCT/KR2023/015299**

(87) International publication number:
**WO 2024/111864 (30.05.2024 Gazette 2024/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.11.2022 KR 20220156610
04.10.2023 KR 20230131707**

(71) Applicant: **Repla Inc.**
**Gyeongsan-si, Gyeongsangbuk-do 38541 (KR)**

(72) Inventors:
• **KIM, Hongrae**
**Suwon-si, Gyeonggi-do 16679 (KR)**
• **CHOI, Donggeon**
**Suwon-si, Gyeonggi-do 16694 (KR)**

(74) Representative: **Maiwald GmbH
Elisenhof
Elisenstraße 3
80335 München (DE)**

(54) ## NOVEL MICROBE WITH PLASTIC DEGRADING ACTIVITY AND USE THEREOF

(57)    The present invention relates to a novel microbe capable of degrading plastic and a method for degrading plastic using same. The microbe can degrade one or more types of plastics selected from the group consisting of polyethylene terephthalate (PET), polyvinyl chloride (PVC), polystyrene (PS), polypropylene (PP), polyurethane (PU), and polyethylene (PE) into low molecular weight substances under appropriate incubation conditions.

[FIG. 5]

EP 4 624 567 A1

## Description

[Technical Field]

The present invention relates to a novel microbe capable of degrading various plastics and a method of degrading plastics using the same.

[Background Art]

The use of plastics has rapidly increased due to their light weight, high physical and chemical durability, high processability, and very low prices, and the amount of plastic production has significantly increased from 234 million tons in 2000 to 460 million tons in 2019. In addition, the amount of plastic waste generated during the same period increased more than two-fold from 156 million tons to 353 million tons (Korea Institute for International Economic Policy (KIEP) World Economic Focus, Vol. 5, No. 13, Current Status and Implications of International Plastic Regulations; published on May 9, 2022).

Due to COVID-19 lockdowns, global plastic usage decreased by 2.2% in 2020 compared to the previous year, but plastic use is increasing again as lockdowns are lifted around the world, particularly in plastic packaging in the fields of healthcare, personal hygiene plastic products, and e-commerce.

The recycling rate of plastic waste worldwide is only 9%, and unrecycled waste plastic is being disposed of through landfill (50%), illegal dumping (22%), and incineration (19%). In particular, plastics account for 80% of marine waste, and marine plastic waste is expected to increase from 9 to 14 million tons per year in 2016 to 23 to 37 million tons per year in 2040. Environmental pollution caused by the influx of plastics not only threatens the ecosystem and human health, but also causes additional costs for waste disposal and recovery from pollution.

Accordingly, research on plastic-degrading microbes is being actively conducted as part of research on methods for treating plastic waste. For example, Korean Patent Publication No. 10-0350928 discloses a novel microorganism, *Klebsiella pneumoniae* CJ-PVA a (Accession No. KFCC-11126), which grows well under aerobic conditions and has improved polyvinyl alcohol degradation ability, and a method of treating wastewater containing polyvinyl alcohol using the same. In addition, Korean Patent Publication No. 10-0513931 discloses *Microbacterium barkeri* LC (Accession No. KCCM 10507) and a method of biologically degrading polyvinyl alcohol using the same.

[Disclosure]

[Technical Problem]

Under the above-described circumstances, the present inventors conducted research to discover a novel plastic-degrading microbe, and isolated a microbe exhibiting plastic-degrading activity after feeding polyethylene to beetle larvae (superworms). As a result of microbial identification, a novel plastic-degrading microbe, an *Acinetobacter guillouiae* repla2 strain, was discovered.

Therefore, an object of the present invention is to provide a novel plastic-degrading microbe and a method of degrading plastics using the same.

[Technical Solution]

In order to achieve the above-described object, one aspect of the present invention provides an *Acinetobacter guillouiae* repla2 strain having plastic-degrading activity, deposited under accession number KACC 81234BP.

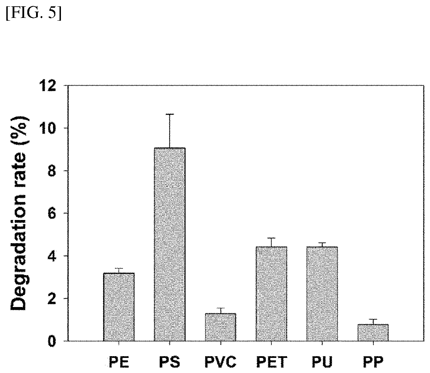

The present inventors isolated an *Acinetobacter guillouiae* repla2 strain from the dominant intestinal microbial community obtained by feeding polyethylene to superworms for two weeks. The isolated *Acinetobacter guillouiae* repla2 strain was capable of degrading various plastics, although the degradation rates varied (FIG. 5).

Therefore, according to one embodiment of the present invention, the plastic is one or more selected from the group consisting of polyethylene terephthalate (PET), polyvinyl chloride (PVC), polystyrene (PS), polypropylene (PP), polyurethane (PU), and polyethylene (PE).

PET is highly transparent, tasteless, and odorless, and thus accounts for most of the plastic beverage bottles on the market. PVC is a type of thermoplastic plastic that is strong, hard or flexible, and it is not easily worn out. PVC is used for artificial leather, packaging materials, pipes, and electrical insulators and has been used for the longest time under the name of vinyl.

PS is a type of thermoplastic plastic that is lightweight, tasteless, and odorless, and is used in household items, toys, electrical insulators, radio and television cases, packaging materials, and the like. PP is manufactured by polymerizing propylene obtained from petroleum and is widely used in bottles and containers.

PE is a type of thermoplastic plastic that is lightweight and flexible, and is a general-purpose plastic widely used from industrial materials to household goods. High-density polyethylene (HDPE) is strong against impacts and has good cold resistance, so it is primarily used to produce shopping bags and pipes. Low-density polyethylene (LDPE) has branches in the polymer, so it has a lower density than linear HDPE, and it is easy to process because it has good elasticity.

In the present invention, "plastic degradation" means degrading a polymer material forming a plastic into a low-molecular weight intermediate material or an intermediate that may be metabolized through a microbial metabolic pathway.

According to one embodiment of the present invention, the plastic degradation may be degrading a polymer material forming a plastic into a hydrocarbon structured material ($[C_nH_n]_n$) having a small molecular weight (Mw ~500).

Another aspect of the present invention provides a method of degrading a plastic, including a step of culturing a plastic with an *Acinetobacter guillouiae* repla2 strain deposited under accession number KACC 81234BP, a strain culture solution, a strain lysate, or a strain-derived plastic-degrading enzyme.

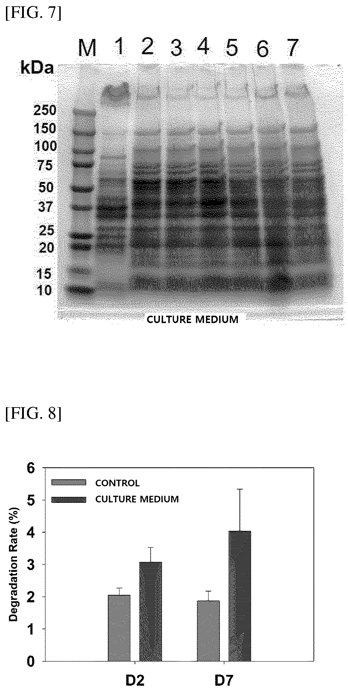

According to an embodiment of the present invention, plastic may be degraded only with a culture solution of the *Acinetobacter guillouiae* repla2 strain (FIG. 8), which means *that Acinetobacter guillouiae* repla2 strain produces and secretes a plastic-degrading enzyme. Therefore, a culture solution of the *Acinetobacter guillouiae* repla2 strain, a strain lysate, or an isolated plastic-degrading enzyme may also be used for plastic degradation.

The culture may be performed at a temperature of 8 to 40 °C for 7 to 60 days after inoculating the *Acinetobacter guillouiae* repla2 strain into a medium containing plastic as the sole carbon source, but is not limited thereto. Preferably, it may be performed at a temperature of 8 to 37 °C for 7 to 30 days.

The medium composition, culture temperature, and culture time used in the plastic degradation method of the present invention may be changed depending on the type of plastic to be degraded, and when the *Acinetobacter guillouiae* repla2 strain and waste plastic or plastic-containing waste are cultured under conditions determined by combining these conditions as process parameters, a specific plastic may be degraded to a relatively large extent or all plastics may be degraded into low-molecular weight materials that can be reused.

The plastic added to the medium may be in the form of finely diced flakes or a thin film (waste vinyl) to increase contact with the *Acinetobacter guillouiae* repla2 strain or a plastic-degrading enzyme secreted by the strain.

Another aspect of the present invention provides a composition for degrading a plastic, including an *Acinetobacter guillouiae* repla2 strain deposited under the accession number KACC 81234BP, a strain culture solution, a strain lysate, or a strain-derived plastic-degrading enzyme.

As described above, the *Acinetobacter guillouiae* repla2 strain expresses a plastic-degrading enzyme and uses plastic as a carbon source, and therefore, the strain itself, the strain culture, the strain lysate, and the strain-derived plastic-degrading enzyme may be effectively used for the purpose of degrading a plastic.

The plastic-degrading enzyme may be an extracellular substance or an intracellular substance of the microbe having plastic-degrading activity and may be mass-produced through various types of genetic recombination.

The *Acinetobacter guillouiae* repla2 strain has a gene that is specifically overexpressed during the plastic-degrading process (FIG. 7), and this gene may be introduced into other microbes through genetic recombination, and the recombinant microbes may be utilized for plastic degradation.

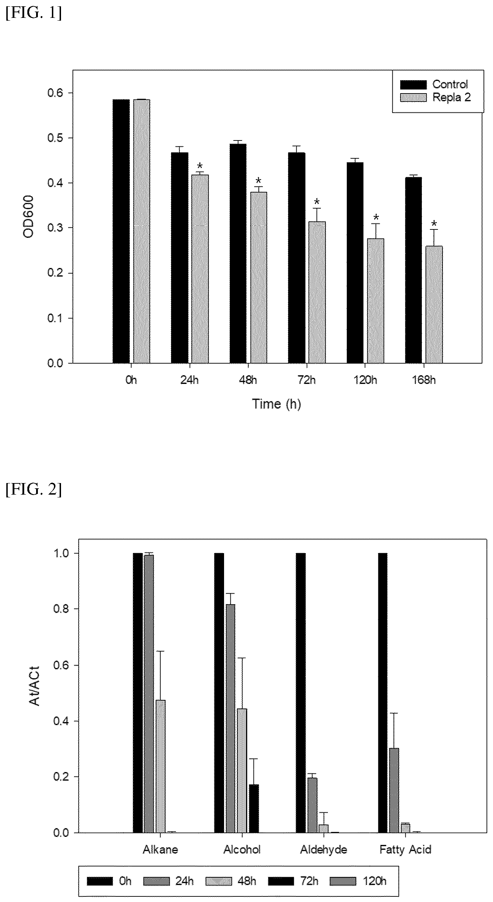

Meanwhile, the *Acinetobacter guillouiae* repla2 strain is capable of degrading various metabolic intermediates (metabolites) of plastics generated during the plastic degradation process (FIG. 2).

Therefore, the present invention provides a composition for degrading a plastic metabolic intermediate, including an *Acinetobacter guillouiae* repla2 strain deposited under the accession number KACC 81234BP, a strain culture solution, a strain lysate, or a strain-derived plastic-degrading enzyme, and a method of degrading a plastic metabolic intermediate.

According to one embodiment of the present invention, the metabolic intermediate of the plastic is one or more selected from the group consisting of an alkane, an alcohol, an aldehyde, and a fatty acid.

In other words, the *Acinetobacter guillouiae* repla2 strain is capable of not only degrading high-molecular weight plastics into low-molecular-weight monomers, but also degrading low-molecular weight monomers into alkanes, alcohols, aldehydes, and fatty acids step by step.

The present inventors also confirmed that the *Acinetobacter guillouiae* repla2 strain hydrophilizes the surface of a plastic film. Therefore, the present invention provides a composition for promoting plastic oxidation, including an *Acinetobacter guillouiae* repla2 strain deposited under accession number KACC 81234BP, a strain culture solution, a strain lysate, or a strain-derived plastic-degrading enzyme, and a method of promoting plastic oxidation.

In the present invention, the composition for degrading a plastic metabolic intermediate, the composition for promoting plastic oxidation may be used alone or in combination with other plastic-degrading microbes/enzymes in a plastic degradation process.

[Advantageous Effects]

The microbe having plastic degradation activity according to the present invention is capable of degrading one or more types of plastic selected from the group consisting of polyethylene terephthalate (PET), polyvinyl chloride (PVC), polystyrene (PS), polypropylene (PP), polyurethane (PU), and polyethylene (PE) and converting them into low-molecular weight substances, and therefore, it can be used in a pretreatment process for plastic degradation and plastic recycling.

[Description of Drawings]

FIG. 1 shows the results of culturing the *Acinetobacter guillouiae* repla2 strain (hereinafter referred to as Repla2) having plastic-degrading activity in a minimal medium containing polyethylene (PE) powder and confirming whether Repla2 metabolizes PE, using 2,6-dichlorophenolindophenol (DCPIP) absorbance: the error bars represent the standard deviation of the mean, and the asterisk represents $p < 0.05$ (Mann-Whitney U test).

FIG. 2 shows the results of culturing Repla2 in a minimal medium containing alkanes, alcohols, aldehydes, or fatty acids as the sole energy source and carbon source and confirming whether Repla2 metabolizes the corresponding substances: At = DCPIP absorbance values over time for the minimal medium containing each energy source and carbon source; Act = DCPIP absorbance values over time for the control group (minimal medium without energy and carbon sources); and error bars = standard deviation of the mean.

FIG. 3 shows the results of culturing a strain (*E. coli*) without plastic-degrading activity and a strain (Ripla2) with plastic-degrading activity in a minimal medium with plastic powder added and then confirming the viable cell counts: the error bars represent the standard deviation of the mean value.

FIG. 4A shows the results of culturing a minimal medium with only a plastic film added without Ripla2 and then confirming the degree of corrosion on the surface of a plastic film.

FIG. 4B shows the results of culturing Ripla2 in a minimal medium with a plastic film added and then confirming the degree of corrosion on the surface of the plastic film.

FIG. 4C shows the results of culturing Ripla2 in a minimal medium with a plastic film added and confirming the formation of a microbial biofilm on the surface of the plastic film.

FIG. 5 shows the results of culturing Ripla2 in a minimal medium containing various types of plastic powder as a sole carbon source and then measuring the plastic degradation rate.

FIG. 6 shows the results of culturing a plastic film with Repla2 and then analyzing the changes in the surface hydrophilicity and chemical functional groups of the plastic film, using X-ray photoelectron spectroscopy (a), Fourier transform infrared spectroscopy (b), and a contact angle meter (c).

FIG. 7 shows the results of confirming the presence of a plastic-degrading enzyme in the culture solution of Repla2 using sodium dodecyl sulfate-polyacrylamide gel electrophoresis (SDS-PAGE).

FIG. 8 shows the results of allowing a culture solution of Repla2 and plastic powder to react for seven days and then confirming the plastic degradation rate. As a control group, the plastic powder was allowed to react in minimal medium for seven days.

[Modes of the Invention]

Hereinafter, one or more embodiments are described in more detail through examples. However, these examples are provided for illustrative purposes only, and the scope of the present invention is not limited to these examples.

**Example 1: Isolation of plastic-degrading microbe**

**1-1. Isolation of plastic-degrading microbe**

By feeding polyethylene (PE) as food to beetle larvae (superworms) for two weeks, selective pressure was applied to hydrocarbon-based substances, which are plastic monomers, to obtain a dominant intestinal microbial community. To isolate only pure intestinal microbes, the larvae were sterilized with 70% ethanol and washed with 0.9% saline. Thereafter, the head and tail of the larvae were removed, and the internal organs were extracted, cut into small pieces with a sterilized knife, and mixed with saline. The epithelial cells and the supernatant were separated by centrifugation.

A part of the separated supernatant was inoculated into a minimal medium, and microbes with potential plastic-degrading activity were cultured under aerobic conditions at 25 °C with shaking at 180 rpm for about 60 days by supplying a PE film as the sole carbon and energy source. The minimal medium composition was as follows: pH 6.51, 0.7 g $NH_2PO_4$, 0.7 g $K_2HPO_4$, 0.7 g $MgSO_4 \cdot 7H_2O$, 1.0 g $NH_4NO_3$, 0.005 g NaCl, 0.002 g $FeSO_4 \cdot 7H_2O$, 0.002 g $ZnSO_4 \cdot 7H_2O$, 0.001 g $MnSO_4 \cdot H_2O$, per 1 L of distilled water. After culture, one strain that grew using the PE film as a carbon source was isolated through a pure isolation method.

**1-2. Microbial identification**

A 16S rRNA base sequence analysis was performed to identify a pure single strain obtained through a third subculture, and the obtained strain was identified to be of the genus *Acinetobacter*. The isolated microorganism was named *Acinetobacter guillouiae* repla2 and deposited in the Korean Agricultural Culture Collection (KACC) of the National Institute of Agricultural Sciences on November 10, 2022, and was assigned the accession number KACC 81234BP.
Hereinafter, the isolated strain is referred to as "Repla2."

**Example 2: Verification of plastic-degrading ability**

**2-1. Verification of direct plastic metabolism ability**

After culturing Repla2 in a medium containing a nutrient medium (Luria-Bertani broth, LB) for 24 hours, cells corresponding to a 600-nm absorbance value of 1 ($10^9$ cells/ml) were collected. The collected Repla2 cells were washed twice with 0.9% saline to remove the previous culture medium and suspended in a minimal medium.
2,6-Dichlorophenolindophenol (DCPIP) as an oxidizing agent and 8 g/L PE (low-density polyethylene, LDPE; Mw ~4,000, Sigma Aldrich) powder were added to the minimal medium. The Repla2 suspension was inoculated into the resulting medium at a ratio of 1/100 ($10^7$ cells/ml) and cultured, and the DCPIP absorbance was measured every 24 hours. Every 24 hours, a part of the culture medium was collected, and the cells and supernatant were separated by centrifugation, and the absorbance (600 nm) of the supernatant was measured. The DCPIP color change of the experimental group was compared with the following two control groups: 1) minimal medium inoculated with only Repla2 (no LDPE powder added); and 2) minimal medium with only LDPE powder added (no Repla2 inoculated).
DCPIP changes its color from blue to colorless when it is reduced by accepting electrons from the energy formed by the metabolic activity of microbes. Through this, the direct plastic-degrading ability and the internal metabolic activity of Ripla2 may be indirectly evaluated.
As a result of measuring the absorbance, it was found that the absorbance of the culture solution decreased in the experimental group with LDPE powder addition + Ripla2 addition as the culture progressed (FIG. 1). This result means that Ripla2 is capable of using LDPE powder as the sole carbon source and energy source, and thus generating reduction energy through internal metabolism. In addition, the absorbance of the culture solution decreased significantly from about one day after the start of the culture, indicating that Ripla2 is capable of degrading LDPE powder from the beginning of the culture.
In FIG. 1, when compared with the control group inoculated with only Ripla2 in the minimal medium, results showing a statistically significant difference are marked with * (Mann-Whitney U test, p <0.05).

**2-2. Verification of internal metabolism of plastic monomers**

In order for microbes to metabolize plastics having polymer structures, the plastics must first be degraded into monomer units with a low molecular weight (Mw ~500), and then the monomers are utilized for microbial metabolism in various forms. Among various monomers, hydrocarbon-structured substances ($[C_nH_n]_n$) are primarily metabolized through the β-oxidation pathway, and metabolism must occur step by step in the order of alkanes, alcohols, aldehydes, and fatty acids (References 2 to 5).
Therefore, the plastic monomer utilization ability of Ripla2 was evaluated.
In the same manner as in Example 2-1, Repla2 was pre-cultured and then suspended in a minimal medium. Alkanes, alcohols, aldehydes, and fatty acids were each added to the minimal medium as the sole energy source and carbon source. The Repla2 suspension was inoculated into the medium at a ratio of 1/100 ($10^7$ cells/ml) and cultured, and the DCPIP absorbance was measured every 24 hours. Every 24 hours, a part of the culture solution was collected, the cells and supernatant were separated by centrifugation, and the absorbance (600 nm) of the supernatant was measured. The DCPIP color change of the experimental group was compared with the following two control groups: 1) No energy source and carbon source added + Repla2 added; and 2) Energy source and carbon source added + Repla2 not added.
As a result of measuring the absorbance, it was found that Repla2 was capable of using all of the alkanes, alcohols, aldehydes, and fatty acids used in the experiment as carbon sources, and the metabolic rate was high in the order of aldehydes, fatty acids, alkanes, and alcohols (FIG. 2).

**2-3. Observation of Ripla2 growth using plastic film as a carbon source**

The plastic-degrading ability of Ripla2 was further demonstrated as described below.

Plastic powder (LDPE) was added to a minimal medium, and *E. coli* (control group) or Ripla2 was inoculated and cultured under aerobic conditions (28 °C, 130 rpm) for approximately seven days. After seven days, the culture solution was collected, and the viable cell counts of *E. coli* and Ripla2 were confirmed as colony-forming units (CFUs).

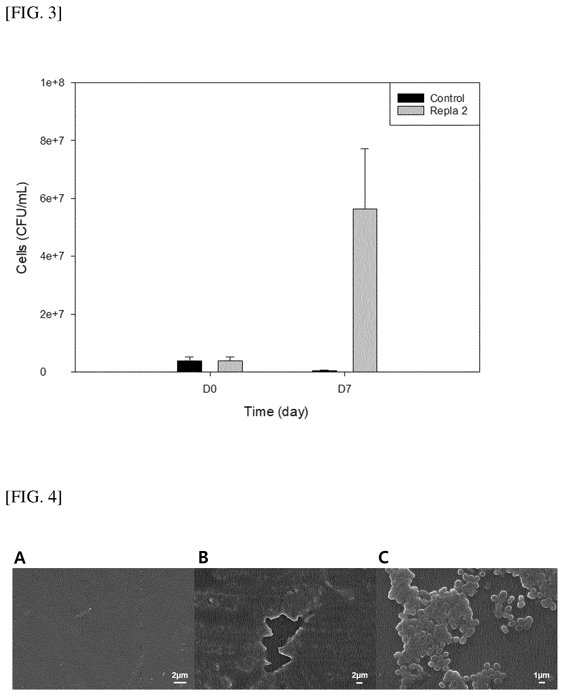

As a result, it was confirmed that *E. coli* died in the control group after seven days of culture, but Ripla2 proliferated in the experimental group inoculated with Ripla2 (FIG. 3). These results suggest that *E. coli* in the control group was not capable of using plastic powder as a carbon source, whereas Ripla2 was capable of using plastic powder as a carbon source.

Next, to observe the biofilm formation by Repla2, a plastic film (LDPE) was added to a minimal medium, Repla2 was inoculated and cultured with shaking under aerobic conditions (28 °C, 130 rpm) for about seven days. After seven days, the plastic film was recovered from the culture medium, and the microbial biofilm formed on the surface of the plastic film and the corrosion due to biodegradation were observed using a scanning electron microscope. As a result, the formation of a microbial biofilm of Repla2 was observed on the plastic surface after culturing for 28 days (FIG. 4C), and corrosion of the plastic surface due to biodegradation was also confirmed (FIG. 4B). On the other hand, in the control group (Repla2 not added), no microbial biofilm formation or plastic surface corrosion was observed (FIG. 4A).

## 2-4. Plastic degradation using Repla2

The plastic-degrading activity of Repla2 was verified using the dry weight measurement method. Plastic powder of various materials was supplied as the sole carbon source in a minimal medium, and Repla2 was inoculated and cultured under aerobic conditions (28 °C, 130 rpm) for seven days with shaking. The plastic degradation rate of the strain was calculated according to Mathematical Formula 1.

[Mathematical Formula 1]

$$\text{Plastic degradation rate of Repla2} = (A-B)/A*100$$

(A: initial plastic weight, B: residual plastic weight after degradation)

As a result of the calculation, it was confirmed that after seven days of culture, 3.18% of PE, 9.07% of PS, 1.29% of PVC, 4.42% of PET, 4.41% of PU, and 0.78% of PP were degraded (FIG. 5).

## 2-5. Plastic oxidation using Repla2

Plastics are hydrophobic materials, and in order to biologically degrade plastics, a process of making the plastics hydrophilic through oxidation must be carried out. Therefore, the plastic oxidation ability of Repla2 was verified by supplying a plastic film as a sole carbon source.

A plastic film (LDPE) was added to a minimal medium, and Repla2 was inoculated and cultured under aerobic conditions (28 °C, 130 rpm) with shaking for about seven days. After seven days, the plastic film was recovered from the culture medium, and the plastic oxidation was confirmed using X-ray photoelectron spectroscopy (XPS), Fourier-transform infrared spectroscopy (FT-IR), and a contact angle meter.

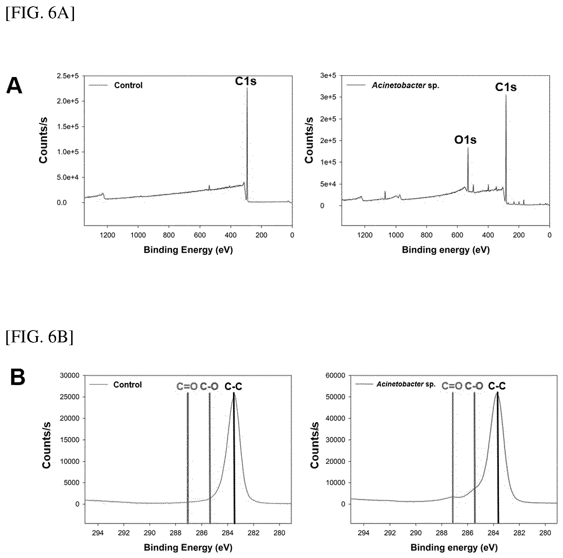

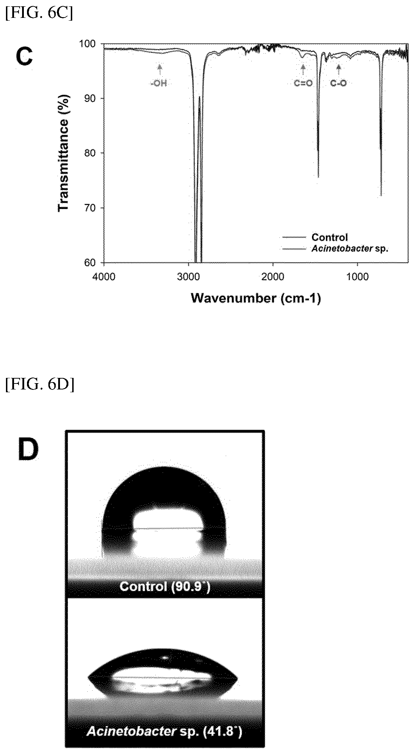

As a result of the XPS analysis, the increase in oxygen elements and the formation of carbonyl groups (C=O) on the plastic surface were confirmed (FIGS. 6A and 6B). The FT-IR analysis results showed that hydroxyl groups (OH) and carbonyl groups (C=O) were formed on the chemical functional groups on the plastic surface (FIG. 6C). In addition, the contact angle measurement confirmed that the contact angle of the plastic cultured with Repla2 decreased, proving that the hydrophilicity of the plastic increased (FIG. 6D).

The results shown in FIG. 6 confirmed that Repla2 is capable of oxidizing plastics. Since plastic oxidation may promote plastic degradation, Repla2 may be utilized not only to degrade plastics through single culture or mixed culture with other strains, but also to increase the plastic degradation rate.

## 2-6. Confirmation of plastic degradation using only enzyme in Repla2 culture medium

After culturing Repla2 with a plastic, microbes were removed through filtering, and the culture medium containing enzymes was obtained. The presence of enzymes in the culture medium was confirmed by sodium dodecyl sulfate-polyacrylamide gel electrophoresis (SDS-PAGE) (FIG. 7).

In addition, the plastic degradation activity of the Repla2 culture medium was verified by dry weight measurement. Plastic powder (LDPE) was added to the Repla2 culture medium from which microbes were removed, and cultured under aerobic conditions (28 °C, 130 rpm) for approximately seven days. The plastic degradation rate by the culture medium was calculated according to Mathematical Formula 1. The plastic degradation rate by the Repla2 culture medium was calculated by subtracting the value shown in the control group from the value shown in the Repla2 culture medium.

As a result of the calculation, it was confirmed that the plastic powder was degraded by 1.02% after two days of culture and by 2.17% after seven days of culture by the Repla2 culture medium (FIG. 8).

[References]

1. Stephen M. Jones, Edward I. Solomon. Electron Transfer and Reaction Mechanism of Laccases. Cell Mol Life Sci. 2015 Mar; 72(5): 869-883.
2. Zahra Montazer et al. Challenges with Verifying Microbial Degradation of Polyethylene. Polymers (Basel). 2020 Jan; 12(1): 123.
3. Zahra Montazer et al. Microbial degradation of low-density polyethylene and synthesis of polyhydroxyalkanoate polymers. Can J Microbiol. 2019 Mar;65(3):224-234.
4. Hector M. Alvarez. Relationship between $\beta$-oxidation pathway and the hydrocarbon-degrading profile in actinomycetes bacteria. International Biodeterioration & Biodegradation Volume 52, Issue 1, July 2003, Pages 35-42.
5. Masaji Watanabe et al. Computational method for analysis of polyethylene biodegradation. Journal of Computational and Applied Mathematics Volume 161, Issue 1, 1 December 2003, Pages 133-144.

[Accession number]

Name of depositor: Korean Agricultural Culture Collection (KACC) of the National Institute of Agricultural Sciences at the Rural Development Administration
Accession number: KACC81234BP
Accession date: 20221104

BUDAPEST TREATY ON THE INTERNATIONAL RECOGNITIO OF THE DEPOSIT OF
MICROORGANISMS FOR THE PURPOSE OF PATENT PROCEDURE

INTERNATIONAL FORM

| TO: Repla CO., LTD.<br>Unit 219, Daehak-ro 280, Gyeongsan-si, Gyeongsangbuk-do | RECEIPT IN THE CASE OF AN ORIGINAL DEPOSIT<br>Issued pursuant to Rule 7.1 |
|---|---|
| I. IDENTIFICATION OF THE MICROORGANISM | |
| Identification reference given by the DEPOSITOR:<br>*Acinetobacter guillouiae* repla2 | Accession number given by the INTERNATIONAL DEPOSITARY AUTHORITY:<br>KACC 81234BP |
| II. SCIENTIFIC DESCRIPTION AND/OR PROPOSED TAXONOMIC DESIGNATION | |
| The microorganism identified under I above was accompanied by:<br>   □ a scientific description<br>   □ a proposed taxonomic designation<br>   (Mark with a cross where applicable) | |
| III. RECEIPT AND ACCEPTANCE | |
| This International Depositary Authority accepts the microorganism identified under I above, which was received by it on November 4, 2022.[1] | |
| IV. RECEIPT OF REQUEST FOR CONVERSION | |
| The microorganism identified under I above was received by this International Depositary Authority on _____ (date of original deposit) and a request to convert the original deposit to a deposit under the Budapest Treaty was received by it on _____ (date of requesting conversion). | |
| V. INTERNATIONAL DEPOSITARY AUTHORITY | |
| Name: Korean Agricultural Culture Collection (KACC) of the National Institute of Agricultural Sciences<br>Address: 166, Nongsaengmyeong-ro, Iseo-myeon, Wanju-gun, Jeollabuk-do, (55365) Republic of Korea | Signature(s) of person(s) having the power to represent the International Depositary Authority or of authorized official(s):<br><br>Date: November 25, 2022 |

1. When Rule 6.4(d) applies, the date shall be the date on which the status of international depositary authority was acquired; when a deposit made under a treaty other than the Budapest Treaty is converted into a deposit under the Treaty after the acquiring of such status, the date shall be the date on which the microorganism was received by the international depositary authority.

Form BP/4

**Claims**

1. An *Acinetobacter guillouiae* repla2 strain having plastic-degrading activity, deposited under accession number KACC 81234BP.

2. The strain of claim 1, wherein the plastic is one or more selected from the group consisting of polyethylene terephthalate (PET), polyvinyl chloride (PVC), polystyrene (PS), polypropylene (PP), polyurethane (PU), and polyethylene (PE).

3. A method of degrading a plastic, comprising a step of culturing a plastic with an *Acinetobacter guillouiae* repla2 strain deposited under accession number KACC 81234BP, a strain culture solution, a strain lysate, or a strain-derived plastic-degrading enzyme.

4. The method of claim 3, wherein the plastic is one or more selected from the group consisting of polyethylene terephthalate (PET), polyvinyl chloride (PVC), polystyrene (PS), polypropylene (PP), polyurethane (PU), and polyethylene (PE).

5. A composition for degrading a plastic, comprising an *Acinetobacter guillouiae* repla2 strain deposited under accession number KACC 81234BP, a strain culture solution, a strain lysate, or a strain-derived plastic-degrading enzyme.

6. The composition of claim 5, wherein the plastic is one or more selected from the group consisting of polyethylene terephthalate (PET), polyvinyl chloride (PVC), polystyrene (PS), polypropylene (PP), polyurethane (PU), and polyethylene (PE).

7. A method of degrading a plastic metabolic intermediate, comprising a step of culturing a plastic metabolic intermediate with an *Acinetobacter guillouiae* repla2 strain deposited under accession number KACC 81234BP, a strain culture solution, a strain lysate, or a strain-derived plastic-degrading enzyme,
   wherein the metabolic intermediate of the plastic is one or more selected from the group consisting of an alkane, an alcohol, an aldehyde, and a fatty acid.

8. A composition for degrading a plastic metabolic intermediate, comprising an *Acinetobacter guillouiae* repla2 strain deposited under accession number KACC 81234BP, a strain culture solution, a strain lysate, or a strain-derived plastic-degrading enzyme,
   wherein the metabolic intermediate of the plastic is one or more selected from the group consisting of an alkane, an alcohol, an aldehyde, and a fatty acid.

9. A composition for promoting plastic oxidation, comprising an *Acinetobacter guillouiae* repla2 strain deposited under accession number KACC 81234BP, a strain culture solution, a strain lysate, or a strain-derived plastic-degrading enzyme,
   wherein the plastic is one or more selected from the group consisting of polyethylene terephthalate (PET), polyvinyl chloride (PVC), polystyrene (PS), polypropylene (PP), polyurethane (PU), and polyethylene (PE).

[FIG. 1]

[FIG. 2]

[FIG. 3]

[FIG. 4]

[FIG. 5]

[FIG. 6A]

[FIG. 6B]

[FIG. 6C]

[FIG. 6D]

EP 4 624 567 A1

[FIG. 7]

CULTURE MEDIUM

[FIG. 8]

15

## INTERNATIONAL SEARCH REPORT

| International application No. |
| --- |
| **PCT/KR2023/015299** |

| A. | CLASSIFICATION OF SUBJECT MATTER |
| --- | --- |

**C12N 1/20**(2006.01)i; **C08J 11/10**(2006.01)i; **B09B 3/60**(2022.01)i; C12R 1/01(2006.01)n

According to International Patent Classification (IPC) or to both national classification and IPC

| B. | FIELDS SEARCHED |
| --- | --- |

Minimum documentation searched (classification system followed by classification symbols)

C12N 1/20(2006.01); C02F 3/34(2006.01); C08J 11/10(2006.01); C12G 1/00(2006.01); C12G 3/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 아시네토박터 길루이에(Acinetobacter guillouiae), 플라스틱(plastic), 분해 (degrading)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WANG, Z. et al. A polystyrene-degrading Acinetobacter bacterium isolated from the larvae of Tribolium castaneum. Science of the Total Environment. 2020, vol. 726, thesis no.:138564, pp. 1-9. See abstract; and pages 5 and 8. | 1-9 |
| A | JP 2020-000104 A (CCI HOLDINGS CO., LTD.) 09 January 2020 (2020-01-09) See entire document. | 1-9 |
| A | KR 10-2022-0091890 A (INDUSTRY-ACADEMIC COOPERATION FOUNDATION GYEONGSANG NATIONAL UNIVERSITY) 01 July 2022 (2022-07-01) See entire document. | 1-9 |
| A | KR 10-2383008 B1 (INDUSTRY FOUNDATION OF CHONNAM NATIONAL UNIVERSITY) 08 April 2022 (2022-04-08) See entire document. | 1-9 |
| A | WO 2018-180187 A1 (AMANO ENZYME INC. et al.) 04 October 2018 (2018-10-04) See entire document. | 1-9 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **09 January 2024** | **10 January 2024** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/015299** |

| C. | DOCUMENTS CONSIDERED TO BE RELEVANT |

| Category* | Citation of document, with indication, where appropriate, of the relevant passages | Relevant to claim No. |
| --- | --- | --- |
| PX | KIM, H. R. et al. Isolation of a polyethylene-degrading bacterium, Acinetobacter guillouiae, using a novel screening method based on a redox indicator. Heliyon. 25 April 2023, vol. 9, thesis no.:e15731, pp. 1-12. See abstract; figure 3; and pages 5 and 9-10. | 1-9 |

Form PCT/ISA/210 (second sheet) (July 2022)

**INTERNATIONAL SEARCH REPORT**
**Information on patent family members**

International application No.

**PCT/KR2023/015299**

| Patent document cited in search report | | | Publication date (day/month/year) | Patent family member(s) | | | Publication date (day/month/year) |
|---|---|---|---|---|---|---|---|
| JP | 2020-000104 | A | 09 January 2020 | JP | 7106370 | B2 | 26 July 2022 |
| KR | 10-2022-0091890 | A | 01 July 2022 | KR | 10-2461894 | B1 | 03 November 2022 |
| | | | | WO | 2022-139548 | A1 | 30 June 2022 |
| KR | 10-2383008 | B1 | 08 April 2022 | WO | 2022-211551 | A1 | 06 October 2022 |
| WO | 2018-180187 | A1 | 04 October 2018 | CN | 111065280 | A | 24 April 2020 |
| | | | | JP | 2020-180187 | A1 | 20 February 2020 |
| | | | | JP | 7138867 | B2 | 20 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 100350928

- KR 100513931

### Non-patent literature cited in the description

- *Current Status and Implications of International Plastic Regulations*, 09 May 2022, vol. 5 (13)
- **STEPHEN M. JONES** ; **EDWARD I. SOLOMON**. Electron Transfer and Reaction Mechanism of Laccases. *Cell Mol Life Sci.*, March 2015, vol. 72 (5), 869-883
- **ZAHRA MONTAZER et al.** Challenges with Verifying Microbial Degradation of Polyethylene. *Polymers (Basel).*, January 2020, vol. 12 (1), 123
- **ZAHRA MONTAZER et al.** Microbial degradation of low-density polyethylene and synthesis of polyhydroxyalkanoate polymers. *Can J Microbiol.*, March 2019, vol. 65 (3), 224-234

- **HECTOR M. ALVAREZ**. Relationship between β-oxidation pathway and the hydrocarbon-degrading profile in actinomycetes bacteria. *International Biodeterioration & Biodegradation*, July 2003, vol. 52 (1), 35-42
- **MASAJI WATANABE et al.** Computational method for analysis of polyethylene biodegradation.. *Journal of Computational and Applied Mathematics*, 01 December 2003, vol. 161 (1), 133-144